# EUROPEAN PATENT APPLICATION

(11) **EP 1 454 608 A1**
(43) Date of publication of application: **08.09.2004**
(21) Application number: 04251206.1
(22) Date of filing: 02.03.2004
(51) Int. Cl.: A61H 23/00, A61H 7/00

(54) **Methods for alleviating symptoms associated with menopause using sensory regimen**

(30) Priority: 03.03.2003 US 378384
(71) Applicant: Johnson & Johnson Consumer Companies, Inc., Skillman, New Jersey 08558-9418 (US)
(72) Inventor: Wiegand, Benjamin, Newton PA 18940 (US); Dean, Kathryn, Ringoes NJ 08551 (US)
(74) Representative: Mercer, Christopher Paul

(57) **Abstract**

The invention relates to a method for alleviating on or more of the symptoms associated with menopause in a woman in the peri-menopause or menopause stage, said method comprising the step of administering a sensory regimen in an amount effective to downregulate the activity of the HPA axis of said woman; wherein said HPA axis comprises:
a) levels of adrenocortical hormone present as a function of time in said woman;
b) a total daily amount of adrenocortical hormone;
c) an integrative measure of morning peak adrenocortical hormone; and
d) an onset of sleep threshold;
wherein said sensory regimen is selected from the group consisting of auditory stimuli, visual stimuli, tactile stimuli, gustatory stimuli, olfactory stimuli, and combinations thereof.

## Description

### BACKGROUND

Menopause is the point in a woman's life when menstruation stops permanently. As a woman transitions to menopause (peri-menopause), she experiences many symptoms including but not limited to hot flashes, night sweats, sleep difficulties, sexual difficulties, vaginal dryness and mild depression. These symptoms are believed to be the result of fluctuating ovarian hormones. In particular, most peri-menopausal and menopausal symptoms are blamed on the changes in estrogen and progesterone levels.

Hot flashes are the most common symptom of menopause. A hot flash starts with a sudden sensation of intense heat often accompanied by sweating and flushing followed by cold shivering as the body tries to compensate for the perceived heat. Hot flashes are believed to occur as a result of decreasing estrogen levels and the subsequent release of hormones that that affect the brain's thermostat. Hot flashes occur sporadically but are thought to be triggered by a number of stimuli such as anxiety, stress, ambient high temperatures, caffeine and alcohol. (Shaw, C., The peri-menopausal hot flash: epidemiology, physiology and treatment, *Nurse Practitioner,* 22(3), pgs. 55-56+61-66, 1997.) Hot flashes often occur during the night and are termed "night sweats" because women will often wake up after the hot flash due to the dampness and discomfort that result from sweating.

Sleep difficulties are also typical symptoms of passing through menopause. Sleep difficulties appear corequisite with hot flashes and night sweats. In addition to hormonal reasons for sleep problems, researchers suspect that many of the menopausal sleep problems are the result of increased stress exposure during this period of a woman's life. Many menopausal women are dealing with the loss of children leaving home and are often responsible for the care of elderly parents.

Hormone replacement therapy (HRT) is a popular treatment option for menopausal symptoms. HRT most commonly consists of supplements of hormones such as estrogen. An alternative to estrogen supplements is progestin in the form of megestrol acetate. While HRT is often effective and is believed to decrease a woman's risk for cardiovascular disease, it has significant drawbacks. Many women are not able to take hormone supplements because of contraindications with other medicines. Also, it is thought that HRT increases the risk for endometrial and breast cancer. HRT does not address the many psychological aspects associated with the intensity of menopausal symptoms such as the increase in stress.

In addition to hormonal supplements, other alternative prescription medications are suggested for the relief of menopausal symptoms including clonidine and methyldopa. This drug works by suppressing the release of norepinephirin which is believed to trigger the brain's thermoregulatory centers. These drugs have had mixed success in clinical trials and are often accompanied with side effects such as fatigue, weakness, dizziness, and nausea. (Shaw, C. 1997)

The use of vitamins and herbals such as vitamin E, ginseng, primrose oil, dong quai, and black cohosh have anecdotal support for the relief of menopausal symptoms, but there is little or no clinical support for their effectiveness.

It is known by those skilled in the art that menopausal symptoms are related to stress and related psychological problems. Stress is known to exacerbate the symptoms associated with menopause including vasometer symptoms (hot flashes, night sweats) and sleep difficulties. One study demonstrated that HRT in combination with psychological treatment was more effective against insomnia than HRT alone. (Anarte MT, Cuadros JL, Herrara J. Hormonal and psychological treatment: Therapeutic alternative for menopausal women? Maturitas 1998; 20: 203.)

Despite these treatments, there remains a need for an alternative non-medical method of alleviating the symptoms associated with menopause for women who do not wish or are unable to take hormonal supplements or homeopathic medicines such as vitamins or herbals. The present invention answers this need.

### SUMMARY OF THE INVENTION

The invention relates to a method for alleviating one or more of the symptoms associated with menopause in a woman in the peri-menopause or menopause stage. The method comprises the step of administering a sensory regimen in an amount effective to downregulate the activity of the HPA axis of said woman; wherein said HPA axis comprises:
a) levels of adrenocortical hormone present as a function of time in said woman;
b) a total daily amount of adrenocortical hormone;

an integrative measure of morning peak adrenocortical hormone; and
an onset of sleep threshold, wherein the activity of the HPA axis can be downregulated by a reduction in at least one of a) through d).

The sensory regimen is selected from the group consisting of auditory stimuli, visual stimuli, tactile stimuli, gustatory stimuli, olfactory stimuli, and combinations thereof.

### DETAILED DESCRIPTION OF THE INVENTION

It has been discovered that one or more of the symptoms associated with menopause can be alleviated by downregulating the activity of the HPA axis of a woman in the peri-menopause or menopause stage where the HPA axis comprises:
a) levels of adrenocortical hormone present as a function of time in said woman;
b) a total daily amount of adrenocortical hormone;
c) an integrative measure of morning peak adrenocortical hormone; and
d) an onset of sleep threshold, wherein the activity of the HPA axis can be downregulated by a reduction in at least one of a) through d).

As used herein, "HPA axis" shall mean the hypothalamus-pituitary-adrenal axis, which is an endocrine system which affects several physiological functions as described by George P. Chrousos and Philip W. Gold in "The Concepts of Stress and Stress System Disorders -- Overview of Physical and Behavioral Homeostasis," JAMA, March 4, 1992, Volume 267, Number 9. Adrenocortical hormones, including cortisol, follow a diurnal rhythym over a 24-hour period with a wakeful period and sleepful period. See, for example, copending U.S. Patent Application Serial No. 10/012,627 filed December 7, 2001, the disclosure of which is hereby incorporated by reference. The area under the curve of the daytime profile can be considered having two distinct areas, the morning peak (referred to herein, as "integrative measure of morning peak adrenocortical hormone")(typically occurring 30 to 45 minutes following waking) and the remaining area under curve. The area under the curve (referred to herein, as "total daily amount of adrenocortical hormone") minus the peak area (integrative measure of morning peak adrenocortical hormone) is a useful index of the activity of the HPA axis. Furthermore, the level of adrenocortical hormone 4 to 8 hours after waking; the level of adrenocortical hormone in the period of time preceding bedtime; and the level of adrenocortical hormone at the onset of sleep threshold are useful indexes of the activity of the HPA axis.

As discussed above, the invention relates to a method for alleviating one or more of the symptoms associated with menopause in a woman in the peri-menopause or menopause stage. The method comprises the step of administering a sensory regimen in an amount effective to downregulate the activity of the HPA axis of said woman; wherein said HPA axis comprises:
a) levels of adrenocortical hormone present as a function of time;
b) a total daily amount of adrenocortical hormone over a 24-hour period;
c) an integrative measure of morning peak adrenocortical hormone; and
d) an onset of sleep threshold.

As used herein, "amount effective" refers to the frequency, level and duration of the sensory regimen sufficient to significantly induce a positive modification in the condition to be treated, but low enough to avoid serious side effects (at a reasonable benefit/risk ratio). The effective amount will vary with the particular condition being treated, the age and physical condition of the patient being treated, the severity of the condition, the frequency, level and duration of the treatment, the nature of concurrent therapy, the specific regimen employed, and like factors. Use of a multiple sensory regimen can affect the duration that would be needed to create the desired response.

As used herein "symptoms associated with menopause" include vasometer symptoms such as hot flashes and/or night sweats, sleep difficulties, mild depression, headaches, vaginal dryness, mood swings, stress and/or irritability. Sleep difficulties include insomnia, night awakenings, daytime fatigue and/or sleep-related disordered breathing.

The sensory regimen is selected from the group consisting of auditory stimuli, visual stimuli, tactile stimuli, gustatory stimuli, olfactory stimuli, and combinations thereof.

The activity of the HPA axis may be downregulated by a reduction in at least one of the following:
a. the average total daily amount of adrenocortical hormone over a 24-hour period in said woman;
b. the average total daily amount adrenocortical hormone minus said integrative measure of morning peak adrenocortical hormone in said woman;
c. the level of adrenocortical hormone in said woman four hours to eight hours after waking;
d. the level of adrenocortical hormone in said woman in the period of time preceding bedtime; and
e. the level of adrenocortical hormone in said woman below said onset of sleep threshold.

Preferably, the average total daily amount of adrenocortical hormone over a 24-hour period in the woman is reduced by at least about 5% to about 50%, more preferably by at least about 10% to about 40%, and most preferably by at least about 15% to about 30%, based on the total daily amount of adrenocortical hormone present in the woman at the start of the regimen.

Preferably, the average total daily amount adrenocortical hormone minus said integrative measure of morning peak adrenocortical hormone over a 24-hour period in the woman is reduced by at least about 5% to about 70%, more preferably by at least about 10% to about 60%, and most preferably by at least about 20% to about 50%, based on the total daily amount of adrenocortical hormone minus said integrative measure of morning peak adrenocortical hormone present in the woman at the start of the regimen.

Preferably, the level of adrenocortical hormone in the woman 4 hours to 8 hours after waking is reduced by at least about 5% to about 70%, more preferably by at least about 10% to about 60%, and most preferably by at least about 20% to about 50%, based on the level of adrenocortical hormone present during that same time period in the woman at the start of the regimen.

Preferably, the level of adrenocortical hormone in the woman in the period preceding bedtime, preferably about 4 hours preceding bedtime, is reduced by at least about 3% to about 50%, more preferably by at least about 5% to about 30%, and most preferably by at least about 5% to about 20%, based on the level of adrenocortical hormone present during that same time period in the woman at the start of the regimen. Preferably, the level of adrenocortical hormone in the period preceding bedtime is reduced to less than 0.3 micrograms/deciliter, more preferably to less than 0.2 micrograms/deciliter, and most preferably to less than 0.15 micrograms/deciliter.

In the method of the invention, the sensory regimen may further include at least one of the following steps selected from the group consisting of:
a. practicing relaxed breathing techniques;
b. hormone replacement therapy; or
c. administering vitamins, herbal supplements, and/or natural extracts, such as, for example, vitamin E, ginseng, primrose oil, dong quai, black cohosh and soy extracts.

Suitable relaxed breathing techniques for use in the present invention include but are not limited to the relaxed breathing described by Dr. Andrew Weil "Breathing: The Master Key to Self Healing" published by Sounds True, Boulder, CO (www.soundstrue.com) 1999. An example includes slow, deep breathing with inhalation of greater than three seconds, a pause between inhalation and exhalation of greater than two seconds, and exhalation of greater than four seconds repeated at least two times.

The combination of the use of the sensory regimen and hormone replacement therapy or the administration of vitamins, herbal supplements and/or thermoregulatory medicines, such as clonidine, methyldopa and mixtures thereof, provides for a more potent treatment and possibly allows for a lower dose of these treatments.

Preferably, the activity of the HPA axis is downregulated within a period of 2 days to 14 days from the start of the sensory regimen according to the invention.

To measure the activity of the HPA axis, cortisol levels in the body, including cortisol found in the serum, saliva or urine, may be measured. Preferably, the cortisol level in saliva is measured because:
(1) collecting saliva is the least stressful, least painful and least invasive;
(2) cortisol levels in saliva are representative of a woman's normal response;
(3) cortisol in saliva is not bound and thus is a more accurate predictor of physiological effect; and
(4) measurement of cortisol in saliva is more instantaneous (less cumulative) relative to other bodily fluids, such as urine.

As described in co-pending US Patent Application Serial No. 10/012,626, filed December 7, 2001, the disclosure of which is hereby incorporated by reference, an enzyme linked immunoassay (ELISA) methodology is useful in the measurement of cortisol at the concentrations typically found in the saliva of a mammal.

The sensory regimen useful in the method of the invention is any regimen that is relaxing to the user. Stimuli used to provide the sensory experience generally are those that provide an experience that the individual who intends to practice the invention finds pleasant. Generally, the sensory regimen is selected from the group consisting of auditory stimuli, visual stimuli, tactile stimuli, gustatory stimuli and olfactory stimuli, and combinations thereof.

Auditory stimuli useful in the method of the invention include, but are but are not limited to, music and sounds of nature that are soothing or relaxing to the user. The term music is used herein to include instrumental and lyrical compositions; tunes; melodies; harmonies; songs; beats and frequencies such as those from metronomes, tuning forks, bells, beat machines, chimes; poetry and rhymes. The music may be of any genre, including, but not limited to, classical, soft rock, easy listening, progressive, country, and show tunes. The sounds of nature include, but are not limited to, animal sounds, such as whales singing or birds chirping; insect sounds, such as crickets; and sounds of the environment, such as a running stream or a waterfall. Sounds that have consistently soft dynamics with minimal melodic and harmonic variability, having little or no conventional beat pitch, little or no vocal, slow tempo, little or no percussion or strong rhythm are particularly effective in relaxing or soothing the user. Sounds that use a binaural beat created by using two pure frequencies, usually one in each ear, are useful in improving the mood of the user. Binaural beats in the frequency range of delta, theta and alpha brain wave frequencies are useful for relaxing the user and beats in the frequency range of beta wave activity are useful for promoting mental alertness in the user. The auditory stimuli may include, but are not limited to, a cassette tape, videotape, compact disc, interactive toys and games, websites, and a computer audio file. An example of suitable relaxing music may be "Night Music 2, HNH International Ltd., 1999."

Visual stimuli useful in the method of the invention include, but are not limited to, soft lights, candles, videos, movies, paintings, murals, books, landscapes, interactive toys and games, websites, and computer image files that are soothing or relaxing to the user. The soft lights may be of any color, such as blue, green, pink, purple, and the like. Cool colors, such as blue and green hues, are preferred to soothe the user and aid relaxation; and warmer colors, such as oranges and reds are preferred to uplift the user. Pastel shades, which are low saturation hues, are useful in soothing the user. The light may be provided in the kit as a bulb, which can be inserted into a lamp at home, or may be provided in the kit as a lamp. Lights that utilize fiber optics may also be useful in the kits of this invention. The fiber optic lights may, as is known in the art, change colors intermittently. Soft lighting of approximately 500 lux is useful in relaxing the user, particularly in the evening hours preceding bedtime. Bright light of around 2000 lux or greater is useful in improving the mood of the user when used in the wakeful period of the day such as at awakening or any other time during the day prior to the few hours preceding bedtime.

Combinations of light and sound that have frequency patterns in the range of delta, theta and alpha brain wave frequencies are useful for relaxing the user and those that have patterns in the frequency range of beta wave activity are useful for promoting mental alertness in the user.

Tactile stimuli useful in the method of the invention include, but are not limited to, computer software, interactive toys and games, bubble baths, lotions, and personal care compositions.

The computer software may be of an interactive nature, such that the consumer relaxes while utilizing the software. Such software includes video games, crossword puzzles and the like.

Gustatory stimuli useful in the method of the present invention include food and beverages, such as, but not limited to, fruits, candies, crackers, cheese, teas, and the like.

Olfactory stimuli useful in the method of the invention include sensory experiences, such as fragrances. Fragrances that the user finds pleasant and have a calming or relaxing effect on their mood are useful in the practice of this invention. Suitable fragrances include fragrances that are perceivable as both pleasant and relaxing to the user, including but not limited to those perfume compositions described in International Patent Application Publication No. WO 02/49600 A1 and available from Quest International. Also suitable are the fragrances described in co-pending U.S. Patent Application Serial No. 09/676,876, filed September 29, 2000 entitled "Method For Calming Human Beings Using Personal Care Compositions," the disclosure of which is incorporated herein by reference. Examples of particularly suitable fragrances include fragrances such as PD 1891, Q-30032 and SG 1443A available from Quest International.

A preferred means of delivering sensory stimuli is in the form of a personal care composition. Personal care compositions are particularly useful in delivering olfactory stimuli. For example, personal care compositions useful in the methods of the invention may then be produced by blending the desired components with the appropriate fragrance using equipment and methodology commonly known in the art of personal care product manufacture. In order to improve the solubilization of the fragrance in aqueous personal care compositions, the fragrance may be pre-blended with one or more of the nonionic surfactants.

Personal care compositions include personal cosmetic, toiletry, and healthcare products such as dry and wet wipes, washes, baths, shampoos, gels, soaps, sticks, balms, mousses, sprays, lotions, creams, cleansing compositions, powders, oils, bath oils and other bath compositions which may be added to a bath. The aforementioned wipes, washes, baths, shampoos, gels, soaps, sticks, balms, mousses, sprays, lotions, creams, cleansing compositions, oils and bath oils are commercially known to those who have a knowledge of preparing personal care compositions. Suitable personal care compositions include, but are not limited to, Johnson's Bedtime Bath® product. In order to achieve the desired response in a mammal, the personal care composition may be used in a dosing amount that is in accordance with the prescribed directions of the personal care composition.

Although a greater effect is generally achieved when multiple stimuli are used together, a single stimuli can also be effective and therefore are included in the invention.

To illustrate the methods of the invention, the following examples are included. These examples do not limit the invention. They are meant only to suggest a method of practicing the invention. Those knowledgeable in the calming of human beings as well as other specialties may find other methods of practicing the invention. Those methods are deemed to be within the scope of this invention.

### EXAMPLE 1: DOWNREGULATION OF ACTIVITY OF HPA AXIS IN MENOPAUSAL AND PERI-MENOPAUSAL WOMEN

Eight menopausal and peri-menopausal women between the ages of 40-60 were recruited to participate in a two-day study to assess the relaxation effect of a sensory regimen. The sensory regimen, hereafter referred to as 'the regimen', consisted of a 15 minute session of practicing a relaxing breathing technique (slow, deep breathing with inhalation of greater than three seconds, a pause between inhalation and exhalation of greater than two seconds, and exhalation of greater than four seconds repeated at least two times) while listening to music (CD - Night Music 2, HNH International Ltd., 1999) and smelling a fragrance rod with relaxing fragrance Q-30032 or SG 1443A, available from Quest International. The participants were provided with the following instructions regarding the fragrance "sniff the fragrance as often as you wish during the designated time period and think about the fragrance and the emotions it brings. Try to picture the scents that compose the fragrance. Re-sniff the fragrance whenever you forget or need to refresh the fragrance image in your mind."

The regimen was compared to a control period consisting of a 15 minute session of reading while sitting quietly in a typical office setting, hereafter referred to as 'the control period'. The subjects were randomized so that half did the regimen on Day 1 and the control period on Day 2. The other half did the control period on Day 1 and the regimen on Day 2. The regimen was shown to have significant relaxation benefits as measured by both questionnaire and saliva measurements. No significant relaxation benefits were observed during the control period.

The questionnaire used in this study assessed the extent to which the woman felt relaxed. The survey was written as follows: "Circle your choice: On a scale of 1-7, how do you feel? 1= Extremely Stressed, 2=Very Stressed, 3=Mildly Stressed, 4=Neither Stressed Nor Relaxed, 5=Mildly Relaxed, 6=Very Relaxed, 7=Extremely Relaxed." The survey question was asked before and after both the regimen and the control period. The survey data from this study is shown in Table 1 below.

**Table 1:**

| **Summary of Survey Data for the Sensory Regimen and Control (Subjects A1-A8)** | | | | | | | | |
|---|---|---|---|---|---|---|---|---|
| | **A1** | **A2** | **A3** | **A4** | **A5** | **A6** | **A7** | **A8** |
| Change in Survey Response During Sensory Regimen | +2 | +2 | +2 | +2 | +2 | +4 | +2 | +2 |
| Change in Survey Response During Control Period | +1 | +1 | +2 | +1 | +0.5 | +2 | 0 | 0 |

As shown in Table 1, on average, subjects indicated that they were 2.3 points more relaxed after the regimen than before. In contrast, subject responses indicate that they were only 1.1 points more relaxed after the control period. This difference was shown to be statistically significant as indicated by a p-value less than 0.05 on a two-tailed t-test.

The regimen was also shown to significantly reduce salivary cortisol concentration thus illustrating down-regulation of the HPA axis. The salivary cortisol data from this study is shown in the Table 2 below.

**Table 2:**

| **Summary of Salivary Cortisol Data for the Sensory Regimen and Control (Subjects A1-A8)** | | | | | | | | |
|---|---|---|---|---|---|---|---|---|
| | **A1** | **A2** | **A3** | **A4** | **A5** | **A6** | **A7** | **A8** |
| Change in Cortisol Concentration (nmol/L) During Sensory Regimen | -2.71 | -0.51 | -7.08 | -2.14 | +0.19 | -5.61 | +0.64 | -1.05 |
| Change in Cortisol Concentration (nmol/L) During Control Period | +3.96 | -1.78 | -1.82 | -0.02 | -0.36 | +0.14 | +0.53 | -2.51 |

### EXAMPLE 2: EFFECT OF SENSORY REGIMEN ON MENOPAUSAL S SYMPTOMS

As shown by Example 1, administration of a sensory regimen according to the invention causes a down-regulation of the activity of the HPA axis. In this Example, four menopausal and peri-menopausal women (B1-B4) suffering from greater than one hot flash per day were recruited to participate in a three-week study to assess the effect of a series of relaxing sensory regimens on one or more of the symptoms of menopause. The series of sensory regimens, hereafter referred to as 'the regimen series', consisted of two daily (morning and afternoon) five minute sessions of listening to relaxing music and smelling a fragrance (Q-30032 or SG 1443A) as well as an evening 15 minute session of practicing a relaxing breathing technique while listening to music (CD - Night Music 2, HNH International Ltd., 1999) and smelling a fragrance (Q-30032 or SG 1443A Quest International Fragrance) each as described in Example 1. In order to control for the use of scented cleansing products, the women were given Johnson's Bedtime Bath and Johnson's pH 5.5 Shower Mousse to use -throughout the study. A fifth participant (B5) did not adhere to the regimen and her data is set forth below as a comparative example.

The women were asked to keep track of their hot flashes using diary sheets that required them to use a stopwatch to measure the duration of their hot flashes. The diary entry sheet also included an anchored questionnaire that assessed various perceived characteristics of the hot flash including heat intensity, duration as compared to previous hot flashes, degree and locations of sweating, and overall hot flash intensity (results in Table 3 below). A "Record of Hot Flashes" sheet was also given to the women so that they could keep track of their daily total number of hot flashes (results in Table 5 below) as well as whether or not they experienced night sweats (results in Table 6 below). Subjects established a baseline of hot flash data during the first week of the study. Materials for the regimen series (fragrance rod and CD) were handed out on Monday of the second week, and subjects practised the regimen series daily for the remaining 14 days.

**Table 3:**

| **Summary Of The Effects Of Sensory Regimen On Hot Flash Characteristics For Subjects B1-B4** | | |
|---|---|---|
| | **% Decrease After 5 days** | **% Decrease After 14 days** |
| **Average # of Daily Hot Flashes** | 24.3* | 26.4 |
| **Average Duration of a Hot Flash** | 14.8 | 10.4 |
| **Average Perceived Duration of a Hot Flash** | 14.9 | 20.7 |
| **Average Perceived Heat Intensity of a Hot Flash** | 22.4 | 28.0* |
| **Average Perceived Overall Intensity of a Hot Flash** | 23.4 | 28.2* |
| **% of Hot Flashes with Sweating** | 18.5 | 17.5 |
| **% of Nights with Night Sweats** | 37.3 | 61.7* |

| | | |
|---|---|---|
| * p<0.05# | | |

**Table 4:**

| **Comparative Example: Summary of the Effects of the Regimen Series on Hot Flash Characteristics (Subject B5)** | | |
|---|---|---|
| | **% Decrease After 5 days** | **% Decrease After 14 days** |
| **Average # of Daily Hot Flashes** | +20% (increase) | 5% |
| **Average Duration of a Hot Flash** | 11% | 18%* |
| **Average Perceived Duration of a Hot Flash** | 7% | 3% |
| **Average Perceived Heat Intensity of a Hot Flash** | 10% | 4% |
| **Average Perceived Overall Intensity of a Hot Flash** | 0% | +8% (increase) |
| **% of Hot Flashes with Sweating** | 45% | +14% (increase) |
| **% of Nights with Night Sweats** | +100% (increase) | +25% (increase) |
| p<0.05 | | |

**Table 5:**

| **Summary of Average Total Daily Hot Flashes Data (Subjects B1-B5)** | | | |
|---|---|---|---|
| **Subject #** | **Average Total Daily Hot Flashes** | | |
| | ***Baseline*** | ***After 5 days of the sensory regimen*** | ***After 10-14 days of the sensory regimen*** |
| **B1** | 3 | 2 | 1 |
| **B2** | 8 | 7 | 6 |
| **B3** | 2 | 1 | 0.6 |
| **B4** | 2 | 0.6 | 0.6 |
| **B5 (comparative)** | 2 | 2 | 2 |

As shown by Table 4, the average number of daily hot flashes for subjects (B1-B4) decreased as a result of doing the daily regimen series. After five days of the regimen, the average number of hot flashes for the subjects decreased by 24.3% and by 36.4% after 10-14 days of the regimen. In contrast, as shown in Table 5, because subject B5 did not complete the regimen, she did not experience any significant reduction in hot flash symptoms.

**Table 6:**

| **Summary of the Percentage of Nights with Night Sweats Data (Subjects B1- B5)** | | | |
|---|---|---|---|
| Subject # | **% of Nights with Night Sweats** | | |
| | *Baseline* | ***After 5 days of the sensory regimen*** | ***After 10-14 days of the sensory regimen*** |
| **B1** | 100 | 20 | 10 |
| **B2*** | 100 | 100 | 100 |
| **B3** | 57 | 60 | 0 |
| **B4** | 30 | 0 | 0 |
| **B5 (Comparative)** | 40 | 60 | 50 |

| | | | |
|---|---|---|---|
| * Subject B2 indicated that even though she continued to have night sweats during the sensory regimen phase, the nights sweats were much milder and she was able to get to sleep much quicker than before doing the sensory regimen. Also, she indicated that she woke up feeling more well-rested. | | | |

As shown by Table 6, the subjects B1-B4 experienced an average 61.7% decrease in the percent of nights with night sweats after 10-14 days of doing the regimen. In contrast, B5 did not experience a decrease in night sweat occurrence.

### EXAMPLE 3: PERCEIVED EFFECTS OF SENSORY REGIMEN ON MENOPAUSE SYMPTOMS

Participants B1-B5 from Example 2 above were asked to respond to a written survey: The results are set forth in Tables 7 and 8 below.

**Table 7:**

| **Summary of Written Survey Responses (Subjects B1-B4)** | | | | | |
|---|---|---|---|---|---|
| | Agree completel y | Agree somewhat | Neither agree nor disagree | Disagree somewhat | Disagree completel y |
| 1. The breathing exercise relaxed me. | 50% | 50% | 0% | 0% | 0% |
| 2. Smelling the relaxing fragrance helped make the relaxation time more beneficial. | 50% | 25% | 25% | 0% | 0% |
| 3. Doing the regimen series made my hot flashes less severe. | 50% | 50% | 0% | 0% | 0% |
| 4. I had less hot flashes during the weeks when I was doing the regimen series. | 50% | 50% | 0% | 0% | 0% |
| 5. I will continue to practice relaxing breathing. | 75% | 25% | 0% | 0% | 0% |
| 6. The "Evening Music for Relaxing Breathing" was effective in relaxing me. | 75% | 25% | 0% | 0% | 0% |
| 7. The regimen series was too time consuming. | 25% | 50% | 0% | 25% | 0% |
| 8. I would be interested in purchasing a product that includes everything I need to practice a regimen series. | 50% | 50% | 0% | 0% | 0% |
| 9. Overall, I enjoyed doing the regimen series. | 50% | 50% | 0% | 0% | 0% |

**Table 8:**

| **Comparative Data: Summary of Subject B5's Written Survey Responses** | |
|---|---|
| | Response |
| 1. The breathing exercise relaxed me. | Agree somewhat |
| 2. Smelling the relaxing fragrance helped make the relaxation time more beneficial. | Disagree completely |
| 3. Doing the regimen series made my hot flashes less severe. | Neither agree nor disagree |
| 4. I had less hot flashes during the weeks when I was doing the regimen series. | Neither agree nor disagree |
| 5. I will continue to practice relaxing breathing. | Agree completely |
| 6. The "Evening Music for Relaxing Breathing" was effective in relaxing me. | Neither agree nor disagree |
| 7. The regimen series was too time consuming. | Agree completely |
| 8. I would be interested in purchasing a product that includes everything I need to practice a regimen series. | Disagree completely |
| 9. Overall, I enjoyed doing the regimen series. | Disagree completely |

In summary, subjects B1-B4 perceived the regimen to be effective in relaxing them and easing their menopause symptoms. This positive response correlates with the recorded reduction in their daily number of hot flashes and night sweat occurrence. In contrast, as shown in Table 8, subject B5 did not notice beneficial effects.

## Claims

1. A sensory regimen, selected from auditory stimuli, visual stimuli, tactile stimuli, gustatory stimuli, olfactory stimuli or a combination thereof, for alleviating one or more of the symptoms associated with menopause in a woman in the peri-menopause or menopause stage, by downregulating the activity of the HPA axis of said woman; wherein said HPA axis comprises:
a) levels of adrenocortical hormone present as a function of time in said woman;
b) a total daily amount of adrenocortical hormone;
c) an integrative measure of morning peak adrenocortical hormone; and an onset of sleep threshold.

2. The sensory regimen according to claim 1, which further comprises a relaxed breathing technique.

3. The sensory regimen according to claim 1 or claim 2, wherein the sensory regimen comprises olfactory stimuli provided by an effective amount of a relaxing fragrance.

4. The sensory regimen according to any one of claims 1 to 3, wherein the sensory regimen comprises visual stimuli provided by soft lighting.

5. The method according to any one of claims 1 to 4, wherein the sensory regimen comprises auditory stimuli provided by relaxing music.

6. The sensory regimen according to any one of claims 1 to 5, wherein the sensory regimen is adapted for administration twice daily for at least one week and comprises olfactory stimuli provided by a relaxing fragrance in combination with auditory stimuli provided by relaxing music.

7. The sensory regimen according to any one of claims 1 to 6, wherein said symptoms are hot flashes, night sweat, sleep difficulties, vaginal dryness, headaches, mood swings, stress, irritability or mild depression.

8. The sensory regimen according to any one of claims 1 to 7, in combination with hormone replacement therapy.

9. The sensory regimen of any one of claims 1 to 8, wherein said down regulation of the activity of the HPA axis is a reduction in at least one of the following:
a. the average total daily amount of adrenocortical hormone in said woman;
b. the average total daily amount adrenocortical hormone minus said integrative measure of morning peak adrenocortical hormone in said woman;
c. the level of adrenocortical hormone in said woman four hours to eight hours after waking;
d. the level of adrenocortical hormone in said woman in the period of time preceding bedtime; and
e. the level of adrenocortical hormone in said woman below said onset of sleep threshold.

10. The sensory regimen of any one of claims 1 to 9, wherein said adrenocortical hormone is cortisol.
